# EUROPÄISCHE PATENTSCHRIFT

(11) **EP 1 468 055 B1**
(45) Veröffentlichungstag und Bekanntmachung des Hinweises auf die Patenterteilung: **20.04.2005**
(21) Anmeldenummer: 03729455.0
(22) Anmeldetag: 14.01.2003
(51) Int. Cl.: C09D 17/00, A61K 7/00, A61K 31/00

(54) **PIGMENTHALTIGE GELMASSE AUF BASIS VON LIPIDEN**
PIGMENT-CONTAINING GEL MASS BASED ON LIPIDS
MATIERE GELIFORME A BASE DE LIPIDES, CONTENANT DES PIGMENTS

(30) Priorität: 16.01.2002 DE 10201370
(43) Veröffentlichungstag der Anmeldung: 20.10.2004
(73) Patentinhaber: Schwan-STABILO Cosmetics GmbH & Co., 90562 Heroldsberg (DE)
(72) Erfinder: SPROGAR, Christian, 91088 Bubenreuth (DE); PINZER, Reinhard, 91220 Schnaittach (DE)
(74) Vertreter: Leissler-Gerstl, Gabriele
(86) Internationale Anmeldenummer: PCT/EP2003/000300
(87) Internationale Veröffentlichungsnummer: WO 2003/060024

(56) Entgegenhaltungen:
- WO-A-00/27350
- DE-U- 29 904 816

## Beschreibung

Die Erfindung betrifft pigmenthaltige Gelmassen auf Basis von Lipiden, diese enthaltende Farbstifte und Verfahren zur Herstellung von pigmenthaltigen Gelmassen und von diese Gelmassen enthaltenden Farbstiften.

Pigmenthaltige Gelmassen auf Basis von Lipiden sind disperse Systeme, die aus einem Gerüst der gelbildenden Komponente mit darin eingelagertem Lipid bestehen. Die Gelmasse enthält außerdem Pigmente zur Anfärbung und ggf. noch in ihr unlösliche Füllstoffe. Derartige Massen sind für das Auftragen von Farben in vielen Bereichen geeignet. Als Hauptanwendungsgebiete für pigmentierte Gelmassen kommen Farbmaterialien zum Zeichnen und Malen und für das Gebiet der dekorativen Kosmetik in Betracht, wo sie in vielen Formen, unter anderem als cremiges Make-up oder als Make-up Stift, als cremige Lidschatten oder als Lidschattenstift, als Rouge, als Lippenstift, als Eyeliner- und als Augenbrauenstift und als Khol- oder Kajalstift anwendbar sind. Insbesondere können derartige pigmenthaltige Gelmassen auf Basis von Lipiden zur Herstellung von Kosmetikstiften verwendet werden.

Zum Zeichnen, Malen oder Schminken verwendete Materialien sollen leicht aufgetragen werden können, sie sollen aber nach dem Auftragen gut haltbar und möglichst wasserfest, im Fall von Kosmetikstiften auch tränenfest, sowie transferresistent sein, d.h. sie sollen nicht auf andere Gegenstände, wie z.B. Gläser oder Textilien abfärben und sie sollen nicht aus dem Auftragsbereich auswandern oder ausbluten.

Bekannte Farbstiftrezepturen basieren auf einem Gemisch aus Fetten, Ölen und Wachsen, welches mit Pigmenten angefärbt wird. So werden in den dem einschlägig befassten Fachmann bekannten Standardwerken beispielsweise Lippen- oder Lidschattenstifte aus natürlichen oder synthetischen Triglyceriden, gehärteten Ölen, Kakaobutter, Cocosfett, natürlichen oder synthetischen Ölen oder Paraffinölen, Siliconölen, natürlichen oder mineralischen Wachsen und üblichen Zusatzstoffen wie Lanolin oder Lanolinderivaten aufgebaute Stiftformulierungen beschrieben. Diese Zubereitungen können ggf. auch noch bekannte kosmetische "Wirkstoffe", z.B. auf Basis von öllöslichen Pflanzenextrakten, Bisabolol oder Vitamine enthalten. In der Regel stellen diese Zubereitungen thixotrope Systeme dar, die sich unter den beim Auftragen auftretenden Scherkräften verflüssigen und so eine sanfte Applikation ermöglichen.

Bekannt ist beispielsweise auch, solche Zubereitungen unter Verwendung flüchtiger Substanzen, wie z.B. kurzkettiger linearer oder cyclischer Siliconöle, dem Fachmann bekannt als Dimethicone oder Cyclomethicone, oder flüchtiger Kohlenwasserstoffe, insbesondere Isoparaffine, oder deren Gemische zu verwenden. Solche Zubereitungen lassen sich besonders sanft auftragen, und nach dem Abdunsten der flüchtigen Bestandteile bleibt ein geschmeidiger Lipidfilm mit transferresistenten Eigenschaften zurück, der zudem nur geringe Tendenzen zum Auswandern in die Feinfältelung der Haut zeigt.

Solche gelartige Zubereitungen wurden im Bereich der Kosmetik bisher überwiegend für Deodorant- und Antiperspirantstifte eingesetzt. Im Allgemeinen haben diese Gele den Vorteil, sich leicht auftragen zu lassen, aber gleichzeitig den Nachteil, dass sie mechanisch nicht belastbar sind. Für Deodorantstifte überwiegt hierbei der Vorteil der leichten Auftragbarkeit, der Stabilität kommt aufgrund der Form dieser Stifte keine wesentliche Bedeutung zu.

Deodorantstifte haben im Verhältnis zu ihrer Länge einen relativ großen Durchmesser und die Anforderungen an die Festigkeit sind aufgrund des Aufbaus der Stifte nicht sehr hoch. Es wurde gefunden, dass sich Massen, die für Deodorantstifte gut geeignet sind, nur sehr bedingt zu dünneren Minen formen lassen und insbesondere, da die Massen zu weich sind, sich nur unter großen Schwierigkeiten oder gar nicht mehr entformen lassen. Insbesondere Minen für Kosmetikstifte weisen im Allgemeinen Durchmesser im Bereich zwischen 2 und 10 mm auf.

Es wurde daher versucht, solche Oleogele unter Verwendung von Wachsen, wie z.B. Bienenwachs, Candelillawachs oder Carnaubawachs herzustellen und zu Minen zu formen. Bienenwachs schrumpft beim Abkühlen und eignet sich daher für Massen, die durch Erhitzen über ihren Erweichungspunkt und Ausgießen in geeignete Formen und Abkühlen zu Stiftrohlingen verarbeitet werden sollen. Candelillawachs und Carnaubawachs geben den Oleogelen Glanz und sind daher bei Lippenstiften und Lidschattenstiften beliebt. Es wurde jedoch gefunden, dass die bekannten Oleogele ein zu geringes Ölbindevermögen aufweisen, so dass es durch Alterung bei längerer Lagerung und bei Temperaturwechseln zu Synäreseeffekten kommen kann, was zu Ölabscheidungen, so genanntem "Schwitzen" bis hin zu völligen Phasentrennungen führt. Das in der Masse enthaltene Öl tritt zunächst in Form von Tröpfchen aus und wandert später in die Umgebung, was die ästhetische Erscheinung des Stiftes beeinträchtigt. Die Stifte verlieren zudem durch das Ausölen ihre Elastizität und werden brüchig. Weiterhin wurde gefunden, dass durch Wegdiffundieren, Verdampfen oder Auswandern des Öls die aus der Masse hergestellten Minen so stark schrumpfen, dass bei einem Versuch zur Herstellung von Farbstiften in Holz eingelegte Minen nach kurzer Zeit aus den Holzhülsen herausrutschten. Werden derartige Minenmassen zur Herstellung von Farbstiften in Kunststoffhülsen oder in Drehmechaniken aus Kunststoff eingegossen, so wurde gefunden, dass auswanderndes Öl an den inneren und äußeren Wandungen entlang spreitet und die Funktion auch dieser Farbstifte beeinträchtigt. Zudem wurde gefunden, dass sehr dünne Minen mit Durchmessern im Bereich um 2 bis 4 mm eine zu geringe Bruchfestigkeit aufweisen, was beim Entnehmen aus - vorzugsweise metallischen ― Gießformen zu erheblichem Bruch und damit zu Ausschuss führt. Wird nach modernen Verfahren direkt in Drehmechaniken eingegossen, so muss bei einem Minenbruch meistens die gesamte Drehmechanik als Ausschuss verworfen werden.

Eine Aufgabe der Erfindung war es daher, die bisher mit Farbminen verbundenen Nachteile zu beseitigen und bekannte Stiftmassen so zu verbessern, dass Farbminen mit weicher Abgabe und einem hohen Ölanteil nach den üblichen Technologien ― wie Extrudieren einer Masse zu Strängen und Einbringen von abgelängten Strangstücken in Holz oder andere geeignete Materialien nach den bei der Bleistiftherstellung bekannten Verfahren, Eingießen einer über den Schmelzpunkt erwärmten Masse in vorzugsweise metallische Gießformen und Einsetzen der nach dem Erkalten erhaltenen Gießlinge in geeignete Applikationsorgane, Eingießen einer über den Schmelzpunkt erwärmten Masse in eine Umhüllung aus einem spitzbaren Material oder Eingießen einer solchen Masse in das Minenführungsteil einer Drehmechanik ― verarbeitet werden können. Insbesondere sollten nach diesen bekannten Verfahren dünne Farbminen mit Durchmessern im Bereich zwischen 2 und 4 mm hergestellt werden können, welche eine ausreichende Zug-, Bruch- und Biegefestigkeit aufweisen.

Eine weitere Aufgabe der Erfindung war es, derartige Farbminen so herzustellen, dass sie bei einem hohen Ölanteil und einer, insbesondere bei kosmetischen Farbminen erwünschten, weichen Abgabe, keine Neigung zum Ausölen zeigen und so, auch noch nach längerer Lagerung, bei wechselnden Temperaturverhältnissen, ein ästhetisches Aussehen behalten.

In DE-OS 199 10 870 wird eine pigmenthaltige Gelmasse auf Ölbasis beschrieben, welche eine Hydroxyfettsäure in Kombination mit einem Alkylmethicone enthält. Derartige Zubereitungen zeichnen sich zwar durch ein gutes Ölbindevermögen aus, die Bruch- und Biegefestigkeit dünner Minen für Farbstifte ist jedoch durchaus noch verbesserungsfähig. Weiterhin beschreibt EP-A 0 861 657 kosmetische Massen, deren Filmbildungseigenschaften und Haftungseigenschaften durch den Zusatz von Ethylcellulose verbessert werden sollen. Es sollen insbesondere gelartige Massen hergestellt werden. DE-OS 19911 748 beschreibt Farbminen, welche in organischen Lösungsmitteln lösliche Alkylcellulose oder Hydroxyalkylcellulose enthalten. Durch diesen Zusatz soll die Zug-, Bruch- und Biegefestigkeit von Farbminen, insbesondere von Farbminen mit dünnem Durchmesser und ― daran gemessen - großer Länge, verbessert werden. Öllösliche Alkylcellulose, insbesondere Ethylcellulose, ist jedoch in den zur Herstellung von Farbminen bekannten Wachsen und Ölen nur schlecht löslich; zudem ist die Einsatzmenge in über den Schmelzpunkt erwärmten Massen, die in diesem Zustand durch Gießen verarbeitet werden sollen, sehr begrenzt, da sie zu einem starken Viskositätsanstieg führen. Die Masse wird leicht zu dickflüssig, um sie mit Erfolg in irgendwelche Öffnungen mit kleinem Durchmesser - nämlich im Bereich der Durchmesser der erwünschten Farbminen (2 bis 4 mm) - eingießen zu können. Auch das aus DE-OS 40 05 894 bekannte Gießverfahren unter Verwendung von beweglichen Füllnadeln führt bei solchen hochviskosen Massen nur bedingt weiter.

Die Extrusion von Massen, die öllösliche Ethylcellulose enthalten, ist grundsätzlich möglich, erfordert jedoch ungewöhnlich hohe Extrusionsdrucke. Die der Erfindung zugrunde liegenden Aufgaben lösen diese Veröffentlichungen somit nicht.

Überraschenderweise wurde nun gefunden, dass eine Kombination von öllöslicher Alkylcellulose, deren Alkylreste geradkettig oder verzweigt sein können und bevorzugt 1 bis 10 Kohlenstoffatome aufweisen, mit einem Alkylgalactomannan Polysaccharide, dessen Alkylrest bevorzugt 1 bis 10 Kohlenstoffatome aufweist und einem Salz, das aus einer langkettigen Fettsäure mit vorzugsweise 16 bis 24 Kohlenstoffatomen und einem Fettsäureamidoalkyldialkylamin einer Fettsäure mit 16 bis 24 Kohlenstoffatomen erhalten wurde, zu Farbminen mit völlig überraschenden Stabilitätseigenschaften führt. Bei letzteren kann es sich beispielsweise um Stearoylamidopropyl Dimethylamine Stearate oder Behenamidopropyl Dimethylamine Stearate oder Stearoylamidopropyl Dimethylamine Behenate oder Behenamidopropyl Dimethylamine Behenate oder um Gemische daraus handeln. Als Alkylcellulose wird bevorzugt Ethylcellulose eingesetzt. Das Alkylgalactomannan Polysaccharide ist bevorzugt ein C1-5 Alkylgalactomannan. Besonders gute Ergebnisse werden erzielt mit der einer Kombination aus öllöslicher Ethylcellulose , einem C1-5 Alkylgalactomannan und Behenamidopropyl Dimethylamine Behenate. Bei den angegebenen Produktbezeichnungen handelt es sich um die dem einschlägig befassten Fachmann bekannten so genannten "INCI-Namen".

C1-5 Alkylgalactomannan und Behenamidopropyl Dimethylamine Behenate sind dem Fachmann als Filmbildner und als viskositätserhöhende Rohstoffe für nicht wässrige Systeme durchaus bekannt. Umso überraschender war daher die Feststellung, dass in der genannten Kombination dieser beiden Rohstoffe mit öllöslicher Alkylcellulose und insbesondere mit Ethylcellulose eine deutlich emiedrigte Viskosität einer über ihren Schmelzpunkt hinaus erwärmten pigmentierten Mischung aus Fetten, Wachsen und Ölen feststellbar war, was zu einer dünnflüssigen, gießfähigen Masse führte. Zudem war es in dieser Mischung möglich - offenbar aufgrund gewisser oberflächenaktiver Effekte des Behenamidopropyl Dimethylamine Behenates - etwas größere Mengen an öllöslicher Ethylcellulose in dieser Masse zu lösen, ohne dass es dabei zu den ohne diesen Zusatz beobachteten Ausfällungen von Ethylcellulose oder zu Viskositätserhöhungen kam. Die Kombination der drei genannten Rohstoffe führte daher zu einer sehr homogenen Mischung mit guter Pigmentverteilung und nach dem Gießen und Abkühlen der Gießlinge zu sehr angenehm und gleichmäßig aufzutragenden Farbminen. Insbesondere wenn solche Farbminen auf die zarte Gesichtshaut im Bereich der Lippen und der Augen aufgetragen werden sollen.

Die Alkylcellulose, bevorzugt eine öllösliche Ethylcellulose, wird dabei in einem Anteil von 0,1 bis 20 Gew.-%, bevorzugt von 0,25 bis 10 Gew.-%, ganz besonders bevorzugt von 0,3 bis 6 Gew.-% eingesetzt. Das Alkylgalactomannan, bevorzugt, das C1-5 Alkylgalactomannan wird in Anteilen von 0,1 bis 20 Gew.-%, bevorzugt von 0,25 bis 10 Gew.-%, ganz besonders bevorzugt von 0,4 bis 4 Gew.-% verwendet. Die Gewichtsprozentangaben beziehen sich dabei jeweils auf das Gewicht der Gelmasse.

Das Salz, das aus einer langkettigen Fettsäure mit vorzugsweise 16 bis 24 Kohlenstoffatomen und einem Fettsäureamidoalkyldialkylamin einer Fettsäure mit 16 bis 24 Kohlenstoffatomen besteht, bevorzugt Behenamidopropyl Dimethylamine Behenate, wird in Anteilen von 0,1 bis 30 Gew.-%, bevorzugt 0,3 bis 20 Gew.-%, besonders bevorzugt 0,5 bis 6 Gew.-% verwendet. Es wurde festgestellt, dass die Eigenschaften der Gelmasse besonders vorteilhaft sind, wenn die eingesetzten Anteile an Alkylcellulose, bevorzugt öllöslicher Ethylcellulose, und Alkylgalactomannan Polysaccharide, C1-5 Alkylgalactomannan in einem Verhältnis von 0,3:1 bis 3:1 zueinander eingesetzt werden und das Verhältnis des Salzes, das aus einer langkettigen Fettsäure mit vorzugsweise 16 bis 24 Kohlenstoffatomen und einem Fettsäureamidoalkyldialkylamin einer Fettsäure mit 16 bis 24 Kohlenstoffatomen besteht, bevorzugt Behenamidopropyl Dimethylamine Behenate, in einem Verhältnis von 0,5:1 bis 5:1 zu der Gesamtmenge der beiden anderen Bestandteile der Kombination steht.

Überraschend war weiterhin, dass die Kombination dieser drei genannten Rohstoffe im abgekühlten Zustand sehr schöne und homogene Gelstrukturen erzeugt, in denen sehr große Mengen einer Ölkomponente stabil gebunden bleiben, so dass es auch nach längerer Zeit nicht zu Separationen oder Ölabscheidungen kommt ― auch dann nicht, wenn die genannten Farbminen bei sehr wechselnden Temperaturen gelagert werden.

Die erfindungsgemäße Gelmasse weist neben der oben beschriebenen Kombination als weiteren mengenmäßig wesentlichen Bestandteil eine Ölkomponente auf. Bestandteile, die die Ölkomponente bilden sind dabei insbesondere Wachse, Öle, flüchtige oder nichtflüchtige Öle, fette Öle, Fette, Paraffine und andere für diese Art von Massen üblicherweise verwendete lipophile Substanzen, sowie deren Mischungen. Bevorzugt enthält die Ölkomponente als wesentliche Bestandteile zumindest ein oder mehrere Wachse und/oder ein oder mehrere Öle bzw. Fette. So kann die Ölkomponente der erfindungsgemäßen Gelmasse pflanzliches, tierisches, mineralisches oder synthetisches Öl und/oder Fett und Wachs umfassen. Für die vorliegende Erfindung sind somit unter anderem Öle, fette Öle, Fette, Paraffine und Vaseline geeignet. Beispielhaft sind hier zu nennen pflanzliche Öle wie z.B. Ricinusöl, Sonnenblumenöl, Sesamöl, Rapsöl, hydrierte pflanzliche Öle wie Cocosöl oder Palmkemöl, Jojobaöl (INCI: Buxus Chinensis) ― im chemischen Sinne ein flüssiges Wachs - Lanolin und Lanolinderivate, Mineralöl, flüchtige Isoparaffine, flüchtige und nicht flüchtige Siliconöle, wie z.B. Cyclomethicone, Dimethicone, Phenyltrimethicone und deren Mischungen. Das Öl wird in einem Anteil von 1 bis 70 Gew.-% verwendet. Unterhalb von 1 Gew.-% ist die Masse auch bei Temperaturen deutlich oberhalb des Schmelzpunktes zu dickflüssig, oberhalb von 70 Gew.-% wird die Gelstruktur nur noch unzureichend ausgebildet und die fertige Farbmine erhält nicht die gewünschten guten Anwendungseigenschaften. Die erwünschte Viskosität der Masse kann durch die Auswahl von Art und Menge der Ölkomponente mit wenigen Routineversuchen in geeigneter Weise eingestellt werden. Ein Teil der Ölkomponente kann auch durch Wachs gebildet werden. Hierbei können pflanzliche, tierische, mineralische und auch synthetische Wachse, wie z.B. Siliconwachse verwendet werden. Bevorzugt werden als Ölkomponente Mischungen aus ölartigen und wachsartigen Rohstoffen eingesetzt. Wenn die Gelmasse zu Farbminen für Kosmetikstifte verwendet werden soll, wird bevorzugt ein in der Kosmetik übliches Wachs eingesetzt, wie z.B. Bienenwachs, Camaubawachs, Candelillawachs, Japanwachs, Ouricouriwachs, Blütenwachse oder Fruchtwachse wie Orangenblütenwachs, Jasminwachs, Apfelwachs oder Orangenwachs, Montanwachs, mikrokristallines Wachs, modifiziertes Bienenwachs wie "Cera Bellina", langkettige Fettalkohole wie Cetylalkohol, Stearylalkohol, Isostearylalkohol, Behenylalkohol, Ester langkettiger Fettalkohole mit langkettigen Fettsäuren wie Cetylpalmitat, Cetearylpalmitat, Stearylstearat, Behenylstearat, C20-40 Alkylstearat oder Mischungen dieser Wachse. Bienenwachs ergibt dabei einen etwas matteren Auftrag, Carnaubawachs und Candelillawachs ergeben einen stärker glänzenden Auftrag. Bevorzugt werden als Ölkomponente oder als Ölanteil der Ölkomponente flüchtige und/oder nicht flüchtige Öle eingesetzt. Besonders bevorzugt sind hierbei Mischungen flüchtiger Siliconöle wie Cyclomethicone und/oder kurzkettige Dimethicone, ggf. auch in Abmischung mit flüchtigen Isoparaffinen wie Isoundecan und/oder Isododecan, mit Wachsen, Fetten, Paraffinen oder fetten Ölen. Die flüchtigen Bestandteile erleichtern dabei das Auftragen der Gelmasse auf die Haut, insbesondere die Gesichtshaut, andererseits erhöhen sie die Haltbarkeit und Wasserfestigkeit resp. Tränenfestigkeit der aufgetragenen Schicht deutlich, wenn die flüchtigen Komponenten abgedampft sind. Bei einer geeigneten Kombination der flüchtigen Komponenten mit der restlichen Ölkomponente kann auch der so genannte "windburn effect" vermieden werden.

Der Anteil von Wachs wird abhängig von der Viskosität der über den Schmelzpunkt erwärmten Gelmasse und den später gewünschten Eigenschaften der fertigen Farbmine ausgewählt. Bevorzugt liegt der Wachsanteil der gesamten Zusammensetzung zwischen 0,1 und 30 Gew.-%.

Der weitere erfindungswesentliche Bestandteil der Gelmasse ist ein Pigment oder eine Mischung von Pigmenten. Hier werden die üblicherweise für pigmentierte Massen eingesetzte Substanzen verwendet, bevorzugt in den üblicherweise eingesetzen Mengen. Für kosmetische Massen werden diese in Deutschland durch die Anlage 3 der Kosmetik-Verordnung geregelt, welche auf der entsprechenden EG-Direktive basiert. Vergleichbare Regelungen bestehen auch in Japan und in den USA. Beispiele für geeignete Pigmente sind anorganische Pigmente wie Titandioxid, Eisenoxide, Ultramarinblau, Chromoxidgrün, Chromoxidhydratgrün, Berliner Blau (Ferric Blue), Glimmer, mit Titanoxid und/oder anderen Metalloxiden beschichtete Glimmer, Verlackungen organischer Färbemittel oder Mischungen daraus. Daneben können noch feinteilige, plättchenförmige Metallpulver, wie Aluminium, Messing, Bronze, Silber oder Gold oder auch feinteilige PET-Plättchen verwendet werden, welche Interferenzerscheinungen zeigen. Bevorzugt werden die für dekorative Kosmetika geeigneten Pigmente eingesetzt. Der Gehalt des Pigments oder der Pigmentmischungen der erfindungsgemäßen Gelmasse liegt in einem Bereich zwischen 0,1 bis 50 Gew.-%, bevorzugt 1 bis 40 Gew.-%, je nach Farbintensität des verwendeten Pigments und der später gewählten Technologie zur Herstellung der fertigen Farbminen. Ganz besonders bevorzugt ist eine Menge von 5 bis 30 Gew.-%.

Zusätzlich zu den aufgeführten Komponenten kann die erfindungsgemäße Gelmasse noch weitere Hilfs- und Zusatzstoffe enthalten, die für derartige Massen üblich oder gebräuchlich sind. Diese weiteren Stoffe werden in den üblicherweise angewendeten Mengen zugegeben. Beispiele hierfür sind Füllstoffe, Verdickungsmittel resp. Viskositätsregulantien, Feuchthaltemittel, Vitamine, Pflanzenextrakte, Emulgatoren, Lösungsvermittler, Dispergierhilfsmittel, Riechstoffe, Aromastoffe, Antioxidantien und Konservierungsmittel, welche in den üblichen Konzentrationen eingesetzt werden können. Bevorzugt ist die erfindungsgemäße Gelmasse wasserfrei, so dass Konservierungsmittel nicht unbedingt erforderlich sind, da Mikroorganismen unter diesen Bedingungen keine geeigneten Lebensbedingungen finden können. Die Verwendung von Antioxidantien ist jedoch bevorzugt, insbesondere dann, wenn die Ölkomponente Bestandteile mit ungesättigten Fettsäuren enthält, um ein Ranzigwerden oder Verderben der erfindungsgemäßen Gelmasse zu verhindern. Zu beachten ist hierbei auch, dass bestimmte Metalloxide, also Pigmente, das Ranzigwerden katalysieren können.

Die Gesamtmenge an Hilfs- und Zusatzstoffen sollte 50 Gew.-% nicht überschreiten und bevorzugt zusammen mit der Menge der Pigmente in einem Bereich von 0,1 bis 50 Gew.-%, bevorzugt 1 bis 45 Gew.-% und besonders bevorzugt in einem Bereich von 5 bis 35 Gew.-% liegen, wobei sich die Prozentangaben jeweils auf die Gelmasse beziehen.

Die erfindungsgemäße pigmentierte Gelmasse auf Ölbasis kann in beliebige Formen gebracht werden. Besonders bevorzugt wird sie zu Minen geformt und dann in Form von Stiften verwendet. Ein weiterer Vorteil der erfindungsgemäßen Gelmasse besteht darin, dass sie mit Gieß- und Extrusionsverfahren geformt werden kann. Besonders bevorzugt wird die erfindungsgemäße Gelmasse zu Farbstiften und Kosmetikstiften verarbeitet. Die aus der erfindungsgemäßen Gelmasse hergestellten Minen haben eine vorteilhafte Gelstruktur, die dazu führt, dass sie sich auch in Form von schlanken, freistehenden Minen ebenso gut auftragen lassen, wie in Form von "gefassten" ― also in Holz oder Kunststoff aufgenommenen - Minen, ohne sich zu verformen oder gar abzubrechen oder zu verschmieren.

Ein weiterer Gegenstand der Erfindung ist ein Farbstift, der eine spitzbare Hülse aus Holz oder einem Holzersatzstoff oder Kunststoff mit einer darin eingebetteten Mine aus einer pigmentierten Gelmasse auf Ölbasis umfasst. Bevorzugt ist der Farbstift ein Kosmetikstift und besonders bevorzugt ist es ein Lippen-, Lidschatten, Lippenkonturen-, Eyeliner-, Khol-, Kajal-, Augenbrauen- oder Abdeckstift, ein so genannter "Concealer".

Der erfindungsgemäße Farbstift wird hergestellt, indem die erfindungsgemäß hergestellte pigmentierte Gelmasse über ihren Schmelzpunkt hinaus erwärmt und in flüssigem Zustand in einen Hülsenrohling eingegossen wird oder indem die pigmentierte Gelmasse extrudiert wird und der so erhaltene Formling, ein Abschnitt eines endlosen Strangs, in genutete Brettchen aus Holz, einem Holzersatzstoff oder aus Kunststoff eingelegt, mit einem weiteren genuteten Brettchen zu Rohlingen verleimt und anschließend nach den bei Bleistiften üblichen Verfahren zu fertigen Stiften weiterverarbeitet wird. Es können zum Beispiel genutete Brettchen mit Vertiefungen zur Aufnahme von jeweils etwa 10 Stiften verwendet werden, wie sie dem Fachmann zur Herstellung von Stiften bekannt sind. In einem weiteren Gießverfahren kann die über ihren Schmelzpunkt erwärmte erfindungsgemäße Gelmasse in Formen eingegossen und nach dem Erkalten ausgeformt und in geeignete Applikationseinheiten eingesetzt werden. Möglich ist es auch, diese Applikationseinheiten mit einem Minenhalteteil auf eine Form aufzustecken und die über ihren Schmelzpunkt erwärmte erfindungsgemäße Gelmasse durch das Minenhalteteil hindurch einzugießen und nach dem Erkalten die fertige, dann fest im Minenhalteteil befindliche Mine, in die Applikationseinheit zurückzudrehen. Dieses Verfahren eignet sich besonders für eine rationelle Fertigung von Kosmetikstiften in hohen Stückzahlen. Bei dieser Herstellungsweise kommen die vorteilhaften Eigenschaften der erfindungsgemäßen Gelmasse bezüglich Zug-, Bruch- und Biegestabilität besonders zum Tragen.

Die aus der erfindungsgemäßen pigmenthaltigen Gelmasse auf Lipidbasis hergestellten Minen haben aufgrund des verbesserten Ölbindevermögens und einer verbesserten Temperaturstabilität sehr positive Eigenschaften. Beispielsweise können sie noch bis etwa 45°C angewendet werden. Die Farbminen haben dann noch eine ausreichende Härte und Stabilität und lassen sich gut spitzen - die Umhüllungen dieser Farbminen lassen sich also leicht in an sich bekannter Weise spanabhebend bearbeiten.

Ein weiterer Gegenstand der Erfindung ist ein Verfahren zur Herstellung von Stiften unter Verwendung der erfindungsgemäßen Gelmasse, wie es in den Ansprüchen 27 und 30 definiert ist.

Aufgrund der Stabilität der Gelmasse kann diese ohne Probleme zu Minen, auch zu sehr dünnen Minen mit einem sehr ungünstigen Verhältnis von Länge zu Durchmesser, verarbeitet werden, z.B. über ihren Schmelzpunkt erwärmt und dann geformt werden. Wesentlich ist dabei, dass die über ihren Schmelzpunkt erwärmte Masse nur eine geringe Viskosität aufweist und somit in Gießverfahren gut zu verarbeiten ist. Da einerseits die Gelmasse sehr homogen und temperaturstabil ist und andererseits die geformten Minen eine ausreichende Festigkeit aufweisen, können die Stifte auch hergestellt werden, indem die erfindungsgemäße Gelmasse direkt in Hülsenrohlinge oder die Minenführungsteile einer Applikationseinheit oder in Gießformen eingegossen werden, auf die eine Drehmechanik aufgesetzt wurde. In diesem Fall wird durch ein für diesen Zweck ausgeformtes Minenhalteteil innerhalb der Drehmechanik hindurchgegossen. Es können aber auch in einem separaten ersten Arbeitsschritt zuerst Minen durch Gießen oder Extrudieren hergestellt werden, und diese dann in die gewünschte Hülse oder Drehmechanik eingesetzt oder nach den bei der Bleistifttechnologie bekannten Verfahren zu Stiften in einer bekannten Umhüllung verarbeitet werden. Diese genannten Varianten bieten Vorteile, und es kann für den jeweiligen Zweck die am besten geeignete Ausführungsform ausgewählt werden.

Gemäß einer Ausführungsform wird zur Herstellung von Minen die pigmentierte Gelmasse durch Gießen oder Extrudieren geformt. Bevorzugt ist ein Formen mit Gießverfahren mit der über den Schmelzpunkt hinaus erwärmten, erfindungsgemäßen Gelmasse, da in diesem Fall die Masse direkt in den zur Aufnahme der Mine vorgesehenen Hülsenrohling aus einem spitzbaren Material eingegossen werden kann. Der Rohling wird dann in an sich bekannten Verfahren zu einem Minenstift weiterverarbeitet. Auf diese Weise lässt sich Ausschuss minimieren.

In einer anderen bevorzugten Ausführungsform wird die über den Schmelzpunkt hinaus erwärmte, erfindungsgemäße Gelmasse in das Minenführungsteil einer Applikationseinheit, vorzugsweise einer Drehmechanik eingegossen und nach dem Erkalten mit den restlichen Komponenten der Applikationseinheit zu einer fertigen Drehmechanik verbunden.

In einer weiteren bevorzugten Ausführungsform wird aus der erfindungsgemäß pigmentierten Gelmasse durch Gießen oder Extrudieren eine Mine geformt und anschließend in eine geeignete Drehmechanik eingesetzt. Die erfindungsgemäße Gelmasse hat auch ohne Abstützung eine so stabile Struktur, dass die in einer Drehmechanik befindliche Mine aus dieser heraus oder in diese hineingedreht werden kann, ohne abzubrechen. Sie eignet sich daher auch sehr gut zur Herstellung von Drehstiften.

Dabei versteht es sich von selbst, dass geeignete Maßnahmen zur sicheren Abdichtung der verwendeten Applikationseinheiten oder Drehmechaniken getroffen werden sollten, die dem Fachmann bekannt sind, wenn die erfindungsgemäße pigmentierte Gelmasse auf Lipidbasis flüchtige Komponenten, wie flüchtige Siliconöle oder Isoparaffine enthält. Werden die nach den vorstehend beschriebenen Verfahren hergestellten Minen nach den in der Bleistiftherstellung bekannten Verfahren weiterverarbeitet, so ist es bevorzugt, die dabei verwendeten, genuteten Brettchen aus Holz oder aus Holzersatzstoffen vorher in geeigneter und bekannter Weise gegen das Hineindiffundieren der flüchtigen Komponenten abzudichten.

Die Erfindung wird durch die nachstehenden Beispiele näher erläutert, ohne sie darauf einzuschränken. Für die Benennung der Rohstoffe wurden die dem Fachmann bekannten INCI-Bezeichnungen verwendet. Die Mengenangaben erfolgen immer in Gewichtsprozent (Gew.-%), bezogen auf das Gesamtgewicht der fertigen Zubereitung.

| **Beispiel 1 ― Eyeliner** | | |
|---|---|---|
| | | Gew.-% |
| (1) | Isostearyl Alcohol | 6,500 |
| (2) | C20-40 Alkyl Stearate | 12,000 |
| (3) | Paraffin | 5,000 |
| (4) | Buxus Chinensis | 2,500 |
| (5) | Ethylcellulose | 2,000 |
| (6) | C1-5 Alkyl Galactomannan | 1,200 |
| (7) | Pigments | 20,000 |
| (8) | Behenamidopropyl Dimethylamine Behenate | 4,750 |
| (9) | Ascorbyl Palmitate | 0,100 |
| (10) | Tocopherol | 0,100 |
| (11) | Cyclomethicone | 45,850 |

Zur Herstellung werden die Komponenten (1) bis (4) zusammengegeben und erhitzt auf etwa 85°C, bis eine flüssige Phase entstanden ist. Darin werden die Komponenten (5) und (6) gelöst und anschließend die Komponente (7) - Pigments - unter Rühren zugesetzt. Die Mischung wird unter Erwärmen flüssig gehalten, dann wird die Komponente (8) zugegeben und in der Mischung unter Rühren gelöst. Nunmehr werden etwa 40% der Komponente (11) unter Rühren zugegeben und die Mischung in geeigneter Weise zur Zerstörung von Pigmentagglomeraten homogenisiert, z.B. mittels eines Dreiwalzenstuhls, Ultra-TURRAX, beheizter Kugelmühle oder dergleichen. Die Mischung wird anschließend wieder über den Schmelzpunkt erwärmt, dabei werden die Komponenten (9) und (10) und der Rest der Komponente (11) zugesetzt und die Gesamtmischung gut durchmischt. Die fertige Mischung wird dann bei etwa 80°C in geeignete Formen eingegossen und nach dem Erkalten geformt und weiterverarbeitet. Man erhält so Farbminen für Eyelinerstifte, mit guter Farbstärke, mit sehr angenehmer Abgabe, welche sehr transferresistent sind und sich nicht auf andere Materialien übertragen und nicht vom Applikationsort auswandern.

| **Beispiel 2 ― Lipliner** | | |
|---|---|---|
| | | Gew.-% |
| (1) | Isostearyl Alcohol | 5,500 |
| (2) | C20-40 Alkyl Stearate | 11,000 |
| (3) | Paraffin | 2,000 |
| (4) | Buxus Chinensis | 2,500 |
| (5) | Ethylcellulose | 1,500 |
| (6) | C1-5 Alkyl Galactomannan | 1,200 |
| (7) | Pigments | 35,000 |
| (8) | Behenamidopropyl Dimethylamine Behenate | 4,750 |
| (9) | Ascorbyl Palmitate | 0,100 |
| (10) | Tocopherol | 0,100 |
| (11) | Cyclomethicone | 36,350 |

Die Herstellung erfolgt in analoger Weise zu Beispiel 1, jedoch wird die Mischung nach Zugabe der Restmenge an Komponente (11) in einem geschlossenen Behältnis abkühlen lassen und in bekannter Weise zu Minen extrudiert. Diese werden in genutete, auf der Innenseite beschichtete Brettchen aus Holz, einem Holzersatzstoff oder aus Kunststoff eingeleimt und in bekannter Weise zu Farbstiften verarbeitet.

Es wurden vier Vergleichsbeispiele angefertigt, in denen die vorgenannte Kombination aus öllöslicher Ethylcellulose, C1-5 Alkylgalactomannan und Behenamidopropyl Dimethylamine Behenate in der Weise variiert wurde, dass jeweils nur zwei dieser Komponenten zur Herstellung eines Lidschattenstiftes gemäß Beispiel 1 verwendet wurden. Die Herstellung erfolgte analog zu Beispiel 1:

| **Vergleichsbeispiele ― Eveliner** | | | | | |
|---|---|---|---|---|---|
| | | (3) Gew.-% | (4) Gew.-% | (5) Gew.-% | (6) Gew.-% |
| (1) | Isostearyl Alcohol | 6,500 | 6,500 | 6,500 | 6,500 |
| (2) | C20-40 Alkyl Stearate | 12,000 | 12,000 | 12,000 | 12,000 |
| (3) | Paraffin | 2,000 | 2,000 | 2,000 | 2,000 |
| (4) | Buxus Chinensis | 2,500 | 2,500 | 2,500 | 2,500 |
| (5) | Ethylcellulose | 2,000 | --- | 2,000 | 2,300 |
| (6) | C1-5 Alkyl Galactomannan | --- | 1,200 | 1,200 | 2,200 |
| (7) | Pigments | 20,000 | 20,000 | 20,000 | 20,000 |
| (8) | Behenamidopropyl Dimethylamine Behenate | 4,750 | 4,750 | --- | --- |
| (9) | Ascorbyl Palmitate | 0,100 | 0,100 | 0,100 | 0,100 |
| (10) | Tocopherol | 0,100 | 0,100 | 0,100 | 0,100 |
| (11) | Cyclomethicone | 50,050 | 50,850 | 55,600 | 54,300 |

**Vergleichsbeispiel 3:** Die Masse wirkt insgesamt inhomogen und bildet schichtartige Strukturen aus. Die Minen sind hart, geben ungleichmäßig ab und brechen sehr leicht. Die über den Schmelzpunkt erwärmte Masse ist hochviskos und schlecht zu gießen, die Viskosität bleibt auch bei weiterer Temperaturerhöhung erhalten. Bei einer Verarbeitung gemäß Beispiel 2 werden beim Extrudieren der Masse spröde Minen erhalten, die sehr leicht brechen.

**Vergleichsbeispiel 4:** Die Masse erstarrt zu sehr weichen Minen, welche sich nur sehr schwer entformen lassen. Die Minenmasse bleibt im Inneren pastös. Die Farbminen sind bei Raumtemperatur schon nicht mehr anwendbar. Bei einer Verarbeitung gemäß Beispiel 2 kann die erkaltete Masse nicht extrudiert werden, da sie als weiche Paste aus dem Extruder fließt. Es sind auf diese Weise keine Farbminen herstellbar, welche akzeptable Gebrauchseigenschaften aufwiesen.

**Vergleichsbeispiel 5:** Die Masse ist hochviskos und schlecht zu vergießen. Die Minen wirken sehr fest und haben eine schlechte und ungleichmäßige Farbabgabe. Es kommt zu starken Separationen von Cyclomethicone an der Oberfläche der Farbmine. Bei einer Herstellung gemäß Beispiel 2 können durch Extrusion keine brauchbaren Farbminen erhalten werden, da die Mehrzahl der Farbminen mehrmals bricht oder sogar zerbröselt.

Vergleichsbeispiel 6: Die Masse ist sehr hochviskos und durch Gießen nicht mehr zu verarbeiten. Eine Temperaturerhöhung bis auf 120°C führt nicht zu einer Erniedrigung der Viskosität. Bei einer Verarbeitung gemäß Beispiel 2 werden durch Extrusion keine brauchbaren Farbminen erhalten. So hergestellte Minen sind hart, brechen sehr leicht und zeigen schlechte und ungleichmäßige Farbabgabe. Es kommt zu starken Separationen von Cyclomethicone auf der Oberfläche dieser Farbminen.

## Patentansprüche

1. Pigmenthaltige Gelmasse auf Basis von Lipiden, **dadurch gekennzeichnet, dass** sie eine Kombination aus
(a) 0,1 bis 20 Gew.-% öllöslicher Alkylcellulose, deren Alkylreste geradkettig oder verzweigt sein können und bevorzugt 1 bis 10 Kohlenstoffatome aufweisen,
(b) 0,1 bis 20 Gew.-% Alkylgalactomannan Polysaccharide, dessen Alkylrest bevorzugt 1 bis 10 Kohlenstoffatome aufweist und
(c) 0,1 bis 30 Gew.-% eines Salzes aus einer langkettigen Fettsäure mit vorzugsweise 16 bis 24 Kohlenstoffatomen und einem Fettsäureamidoalkyldialkylamin
enthält.

2. Gelmasse nach Anspruch 1, **dadurch gekennzeichnet, dass** die öllösliche Alkylcellulose öllösliche Ethylcellulose ist.

3. Gelmasse nach Anspruch 1, **dadurch gekennzeichnet, dass** das Alkylgalactomannan Polysaccharide C1-5 Alkylgalcatomannan ist.

4. Gelmasse nach Anspruch 1, **dadurch gekennzeichnet, dass** das Salz aus einer langkettigen Fettsäure und einem Fettsäurealkylamidodialkylamin Stearylamidopropyl Dimethylamine Stearate, Behenamidopropyl Dimethylamine Stearate oder Stearoylamidopropyl Dimethylamine Behenate oder Behenamidopropyl Dimethylamine Behenate oder ein Gemisch aus diesen Salzen ist.

5. Gelmasse nach Anspruch 1, **dadurch gekennzeichnet, dass** das Salz ein Behenamidopropyl Dimethylamine Behenate ist.

6. Gelmasse nach Anspruch 1, **dadurch gekennzeichnet, dass** sie eine Kombination aus
(a) 0,1 bis 20 Gew.-% öllöslicher Ethylcellulose,
(b) 0,1 bis 20 Gew.-% C1-5 Alkylgalactomannan und
(c) 0,1 bis 30 Gew.-% Behenamidopropyl Dimethylamine Behenate
enthält.

7. Gelmasse nach Anspruch 1, **dadurch gekennzeichnet, dass** sie eine Kombination aus
(a) 0,25 bis 10 Gew.-% öllöslicher Ethylcellulose,
(b) 0,25 bis 10 Gew.-% C1-5 Alkylgalactomannan und
(c) 0,30 bis 20 Gew.-% Behenamidopropyl Dimethylamine Behenate
enthält.

8. Gelmasse nach Anspruch 1, **dadurch gekennzeichnet, dass** sie eine Kombination aus
(a) 0,4 bis 4 Gew.-% öllöslicher Ethylcellulose,
(b) 0,4 bis 4 Gew.-% C1-5 Alkylgalactomannan und
(c) 0,5 bis 6 Gew.-% Behenamidopropyl Dimethylamine Behenate
enthält.

9. Gelmasse nach einem der vorhergehenden Ansprüche, **dadurch gekennzeichnet, dass** die öllösliche Ethylcellulose und das C1-5 Alkylgalactomannan in einem Verhältnis von 0,3 : 1 bis 3 : 1 zueinander eingesetzt werden.

10. Gelmasse nach einem der vorhergehenden Ansprüche, **dadurch gekennzeichnet, dass** das Behenamidopropyl Dimethylamine Behenate in einem Verhältnis von 0,5 : 1 bis 5 : 1 zu der Gesamtmenge der beiden anderen Bestandteile der Kombination steht.

11. Gelmasse nach einem der vorhergehenden Ansprüche, **dadurch gekennzeichnet, dass** die Ölkomponente pflanzliches, tierisches, mineralisches oder synthetisches Öl und/oder Fett, nichtflüchtiges und/oder flüchtiges Siliconöl und Wachs umfaßt.

12. Gelmasse nach Anspruch 11, **dadurch gekennzeichnet, dass** das pflanzliche, tierische, mineralische oder synthetische Öl und/oder nichtflüchtige oder flüchtige Siliconöl in einem Anteil von 1 bis 70 Gew.-%, bezogen auf das Gesamtgewicht der Zusammensetzung, enthalten ist.

13. Gelmasse nach Anspruch 12, **dadurch gekennzeichnet, dass** als Öl und/oder Fett Ricinusöl, Sonnenblumenöl, Sesamöl, Rapsöl, hydriertes Cocosöl, hydriertes Palmkernöl, Jojobaöl, Mineralöl, Paraffin, Vaseline, Lanolin oder Lanolinderivate, flüchtiges Isoparaffin, Cyclomethicone, Dimethicone, Phenyltrimethicone oder ein Gemisch aus den genannten Ölen enthalten ist.

14. Gelmasse nach einem der vorhergehenden Ansprüche, **dadurch gekennzeichnet, dass** sie Wachs in einem Anteil von 0,1 bis 30 Gew.-%, bezogen auf das Gesamtgewicht der Zusammensetzung, enthält.

15. Gelmasse nach Anspruch 14, **dadurch gekennzeichnet, dass** sie als Wachs ein natürliches, mineralisches oder synthetisches Wachs enthält.

16. Gelmasse nach Anspruch 15, **dadurch gekennzeichnet, dass** sie als Wachs Bienenwachs, modifiziertes Bienenwachs (sog. "Cera Bellina"), Carnaubawachs, Candelillawachs, Japanwachs, Ouricouriwachs, Blütenwachs, Orangenblütenwachs, Jasmainwachs, Fruchtwachs, Apfelwachs, Orangenwachs, Montanwachs, mikrokristallines Wachs, langkettige Fettalkohole, Ester aus langkettigen Fettalkoholen und langkettigen Fettsäuren oder Mischungen aus den genannten Wachsen enthält.

17. Gelmasse nach einem der vorhergehenden Ansprüche, **dadurch gekennzeichnet, dass** es sich bei dem langkettigen Fettalkohol um Cetylalkohol, Stearylalkohol, Behenylalkohol oder deren Gemische handelt.

18. Gelmasse nach einem der vorhergehenden Ansprüche, **dadurch gekennzeichnet, dass** es sich bei dem Ester aus langkettigem Fettalkohol und langkettiger Fettsäure um Cetylpalmitat, Cetearylpalmitat, Stearylstearat, Behenylstearat, C 20-40 Alkylstearat oder deren Gemische handelt.

19. Gelmasse nach einem der vorhergehenden Ansprüche, **dadurch gekennzeichnet, dass** sie zusätzlich Hilfs- und Zusatzstoffe in einem Anteil von 0,1 bis 50 Gew.-%, bezogen auf das Gesamtgewicht der Zusammensetzung, enthält.

20. Gelmasse nach einem der vorhergehenden Ansprüche, **dadurch gekennzeichnet, dass** sie Pigmente in einer Menge von 0,1 bis 50 Gew.-%, bevorzugt 1 bis 45 Gew.-%, ganz besonders bevorzugt 5 bis 35 Gew.-%, bezogen auf das Gesamtgewicht der Zusammensetzung, enthält.

21. Gelmasse nach einem der vorhergehenden Ansprüche, **dadurch gekennzeichnet, dass** als Pigmente Titandioxid, Eisenoxide, Ultramarinblau, Chromoxidgrün, Chromoxidhydratgrün, Berliner Blau, Glimmer, Glanzpigmente, plättchenförmige Metallpulver, feinteilige PET-Plättchen, Verlackungen organischer Färbemittel oder deren Mischungen enthalten sind.

22. Gelmasse nach einem der vorhergehenden Ansprüche, **dadurch gekennzeichnet, dass** sie in Form einer gegossenen oder extrudierten Mine vorliegt.

23. Farbstift, umfassend eine Mine aus einer Gelmasse nach einem der Ansprüche 1 bis 22, die von einem Hülsenrohling aus Holz, einem Holzersatzstoff oder Kunststoff umgeben ist.

24. Farbstift, umfassend eine Mine aus einer Gelmasse nach einem der Ansprüche 1 bis 22, die in die Drehmechanik eines Drehstiftes eingesetzt oder eingegossen ist.

25. Farbstift nach einem der Ansprüche 23 oder 24, **dadurch gekennzeichnet, dass** es ein Kosmetikstift ist.

26. Farbstift nach einem der Ansprüche 23 oder 24, **dadurch gekennzeichnet, dass** es ein Lippen-, Lippenkonturen-, Lidschatten-, Eyeliner-, Khol-, Kajal-, Augenbrauenoder Abdeckstift (sog. "Concealer") ist.

27. Verfahren zur Herstellung eines Farbstiftes, **dadurch gekennzeichnet, dass** eine Gelmasse nach einem der Ansprüche 1 bis 22 aufgeschmolzen wird und anschließend in einen Hülsenrohling für einen Minenstift aus Holz, einem Holzersatzstoff oder aus Kunststoff eingegossen wird und dann zu einem Stift verarbeitet wird.

28. Verfahren zur Herstellung eines Farbstiftes, **dadurch gekennzeichnet, dass** eine Gelmasse nach einem der Ansprüche 1 bis 22 extrudiert, zu Stücken von Farbminen abgelängt und in genutete Brettchen aus Holz, einem Holzersatzstoff oder Kunststoff eingeleimt und dann zu einem Stift weiterverarbeitet wird.

29. Verfahren zur Herstellung eines Farbstiftes, **dadurch gekennzeichnet, dass** zur Herstellung einer Drehmechanik eine Gelmasse nach einem der Ansprüche 1 bis 22 aufgeschmolzen wird und durch ein auf eine Gießform aufgesetztes Minenhalteteil einer Drehmechanik gegossen wird und die nach dem Erkalten gebildete Mine nach dem Entformen in die Drehmechanik eines Drehstiftes hineingedreht wird.

30. Verfahren zur Herstellung eines Farbstiftes, **dadurch gekennzeichnet, dass** zur Herstellung einer Drehmechanik eine Gelmasse nach einem der Ansprüche 1 bis 22 aufgeschmolzen wird und in eine Gießform eingegossen wird und die nach dem Erkalten gebildete Mine in das Minenhalteteil einer Drehmechanik eines Drehstiftes eingesetzt wird.

## Claims

1. Pigment-bearing gel material based on lipids, **characterised in that** it includes a combination of:
(a) between 0.1 and 20% by weight of oil-soluble alkylcellulose whose alkyl residues can be straight-chain or branched and preferably have between 1 and 10 carbon atoms,
(b) between 0.1 and 20% by weight of alkylgalactomannan polysaccharide whose alkyl residue preferably has between 1 and 10 carbon atoms, and
(c) between 0.1 and 30% by weight of a salt from a long-chain fatty acid with preferably between 16 and 24 carbon atoms and a fatty acid amidoalkyldialkylamine.

2. A gel material as set forth in claim 1 **characterised in that** the oil-soluble alkylcellulose is oil-soluble ethylcellulose.

3. A gel material as set forth in claim 1 **characterised in that** the alkylgalactomannan polysaccharide is C1-5 alkylgalactomannan.

4. A gel material as set forth in claim 1 **characterised in that** the salt from a long-chain fatty acid and a fatty acid alkylamidodialkylamine is stearylamidopropyl dimethylamine stearate, behenamidopropyl dimethylamine stearate or stearoylamidopropyl dimethylamine behenate or behenamidopropyl dimethylamine behenate or a mixture of those salts.

5. A gel material as set forth in claim 1 **characterised in that** the salt is a behenamidopropyl dimethylamine behenate.

6. A gel material as set forth in claim 1 **characterised in that** it contains a combination of:
(a) between 0.1 and 20% by weight of oil-soluble ethylcellulose,
(b) between 0.1 and 20% by weight of C1-5 alkylgalactomannan and
(c) between 0.1 and 30% by weight of behenamidopropyl dimethylamine behenate.

7. A gel material as set forth in claim 1 **characterised in that** it preferably contains a combination of:
(a) between 0.25 and 10% by weight of oil-soluble ethylcellulose,
(b) between 0.25 and 10% by weight of C1-5 alkylgalactomannan and
(c) between 0.30 and 20% by weight of behenamidopropyl dimethylamine behenate.

8. A gel material as set forth in claim 1 **characterised in that** it quite particularly preferably contains a combination of:
(a) between 0.4 and 4% by weight of oil-soluble ethylcellulose,
(b) between 0.4 and 4% by weight of C1-5 alkylgalactomannan and
(c) between 0.5 and 6% by weight of behenamidopropyl dimethylamine behenate.

9. A gel material as set forth in one of the preceding claims **characterised in that** the oil-soluble ethylcellulose and the C1-5 alkylgalactomannan are used in a ratio of between 0.3:1 and 3:1 relative to each other.

10. A gel material as set forth in one of the preceding claims **characterised in that** behenamidopropyl dimethylamine behenate is in a ratio of between 0.5:1 and 5:1 to the total amount of the other two constituents of the combination.

11. A gel material as set forth in one of the preceding claims **characterised in that** the oil component includes vegetable, animal, mineral or synthetic oil and/or fat, non-volatile and/or volatile silicone oil and wax.

12. A gel material as set forth in claim 11 **characterised in that** the vegetable, animal, mineral or synthetic oil and/or non-volatile or volatile silicone oil is contained in a proportion of between 1 and 70% by weight with respect to the total weight of the composition.

13. A gel material as set forth in claim 12 **characterised in that** the oil and/or fat contained therein is castor oil, sunflower oil, sesame seed oil, rapeseed oil, hydrated coconut oil, hydrated palm oil, jojoba oil, mineral oil, paraffin, petroleum jelly, lanolin or lanolin derivatives, volatile isopraffin, cyclomethicone, dimethicone, phenyltrimethicone or a mixture of said oils.

14. A gel material as set forth in one of the preceding claims **characterised in that** it contains wax in a proportion of between 0.1 and 30% by weight with respect to the total weight of the composition.

15. A gel material as set forth in claim 14 **characterised in that** as wax it contains a natural, mineral or synthetic wax.

16. A gel material as set forth in claim 15 **characterised in that** as wax it contains beeswax, modified beeswax (so-called 'Cera Bellina'), carnauba wax, candelilla wax, Japan wax, ouricuri wax, flower wax, orange flower wax, jasmine wax, fruit wax, apple wax, orange wax, lignite wax, microcrystalline wax, long-chain fatty alcohols, esters of long-chain fatty alcohols and long-chain fatty acids or mixtures of said waxes.

17. A gel material as set forth in one of the preceding claims **characterised in that** the long-chain fatty alcohol is cetylalcohol, stearylalcohol, behenylalcohol or mixtures thereof.

18. A gel material as set forth in one of the preceding claims **characterised in that** the ester of long-chain fatty alcohol and long-chain fatty acid is cetylpalmitate, cetearylpalmitate, stearylstearate, behenylstearate, C20-40 alkylstearate or mixtures thereof.

19. A gel material as set forth in one of the preceding claims **characterised in that** it additionally includes auxiliary substances and additives in a proportion of between 0.1 and 50% by weight with respect to the total weight of the composition.

20. A gel material as set forth in one of the preceding claims **characterised in that** it contains pigments in an amount of between 0.1 and 50% by weight, preferably between 1 and 45% by weight, quite particularly preferably between 5 and 35% by weight with respect to the total weight of the composition.

21. A gel material as set forth in one of the preceding claims **characterised in that** the pigments contained therein are titanium dioxide, iron oxides, ultramarine blue, chromium oxide green, chromium hydroxide green, Berlin Blue, mica, sheen pigments, flake-form metal powders, finely divided PET flakes, lakes of organic coloring agents or mixtures thereof.

22. A gel material as set forth in one of the preceding claims **characterised in that** it is in the form of a cast or extruded lead.

23. A color pencil including a lead comprising a gel material as set forth in one of claims 1 through 22 which is surrounded by a sheath blank of wood, a wood substitute or plastic material.

24. A color pencil including a lead comprising a gel material as set forth in one of claims 1 through 22 which is fitted or cast into the rotary mechanism of a rotary pencil.

25. A color pencil as set forth in one of claims 23 and 24 **characterised in that** it is a cosmetic pencil.

26. A color pencil as set forth in one of claims 23 and 24 **characterised in that** it is a lipstick, lipliner pencil, eyeshadow pencil, eyeliner pencil, kohl pencil, kajal pencil, eyebrow pencil or concealing pencil (so-called 'concealer').

27. A process for the production of a color pencil **characterised in that** a gel material as set forth in one of claims 1 through 22 is melted and then cast into a sheath blank for a lead-bearing pencil consisting of wood, a wood substitute or plastic and is then processed to form a pencil.

28. A process for the production of a color pencil **characterised in that** a gel material as set forth in one of claims 1 through 22 is extruded, cut to length to form portions of color leads, glued into grooved board portions of wood, a wood substitute or plastic material and then subjected to further processing to form a pencil.

29. A process for the production of a color pencil **characterised in that** to produce a rotary mechanism a gel material as set forth in one of claims 1 through 22 is melted and poured through a lead-holding portion, which is fitted on to a casting mold, of a rotary mechanism, and the lead formed after cooling, after removal from the mold, is rotated into the rotary mechanism of a rotary pencil.

30. A process for the production of a color pencil **characterised in that** to produce a rotary mechanism a gel material as set forth in one of claims 1 through 22 is melted and poured into a casting mold and the lead formed after cooling, is fitted into the lead-holding portion of a rotary mechanism of a rotary pencil.

## Revendications

1. Masse géliforme à base de lipides et contenant des pigments, **caractérisée en ce qu'**elle comprend une combinaison de :
a) 0,1 à 20% en poids d'une alkylcellulose soluble dans les huiles, et dont les restes alkyle peuvent être à chaîne droite ou ramifiée, et présentant de préférence de 1 à 10 atomes de carbone,
b) 0,1 à 20% en poids d'un polysaccharide du type alkylgalactomannane dont les restes alkyle présentent de préférence de 1 à 10 atomes de carbone, et
c) 0,1 à 30% en poids d'un sel d'un acide gras à longue chaîne contenant de préférence de 16 à 24 atomes de carbone et d'une amidoalkyldialkylamine d'un acide gras.

2. Masse géliforme selon la revendication 1, **caractérisée en ce que** l'alkylcellulose soluble dans les huiles est l'éthylcellulose.

3. Masse géliforme selon la revendication 1, **caractérisée en ce que** le polysaccharide du type alkylgalactomannane est un alkylgalactomannane en C1-5.

4. Masse géliforme selon la revendication 1, **caractérisée en ce que** le sel d'un acide gras à longue chaîne et d'une alkylamidodialkylamine d'acide gras est le stéarate de stéarylamidopropyldiméthylamine, le stéarate de béhènamidopropyldiméthylamine ou le béhènate de stéaroylamidopropyldiméthylamine ou le béhènate de béhènamidopropyldiméthylamine, ou un mélange de ces sels.

5. Masse géliforme selon la revendication 1, **caractérisée en ce que** le sel est le béhènate de béhènamidopropyldiméthylamine.

6. Masse géliforme selon la revendication 1, **caractérisée en ce qu'**elle comprend :
a) 0,1 à 20% en poids d'éthylcellulose soluble dans les huiles,
b) 0,1 à 20% en poids d'alkylgalactomannae en C1-5 et
c) 0,1 à 30% en poids de béhènate de béhènamidopropyldiméthylamine.

7. Masse géliforme selon la revendication 1, **caractérisée en ce qu'**elle comprend :
a) 0,25 à 10% en poids d'éthylcellulose soluble dans les huiles,
b) 0,25 à 10% en poids d'alkylgalactomannane en C1-5, et
c) 0,30 à 20% en poids de béhènate de béhènamidopropyldiméthylamine.

8. Masse géliforme selon la revendication 1, **caractérisée en ce qu'**elle comprend une combinaison de :
a) 0,4 à 4% en poids d'éthylcellulose soluble dans les huiles,
b) 0,4 à 4% en poids d'alkylgalactomannane en C1-5 et
c) 0,5 à 6% en poids de béhènate de béhènamidopropyldiméthylamine

9. Masse géliforme selon l'une quelconque des revendications précédentes, **caractérisée en ce que** l'éthylcellulose soluble dans les huiles et l'alkylgalactomannane en C1-5 sont introduits dans une proportion de 0,3 :1 à 3 :1 l'un par rapport à l'autre.

10. Masse géliforme selon l'une quelconque des revendications précédentes, **caractérisée en ce que** le béhènate de béhènamidopropyldiméthylamine est présent à raison de 0,5 :1 à 5 :1 par rapport à la quantité totale des deux autres constituants de la combinaison.

11. Masse géliforme selon l'une quelconque des revendications précédentes, **caractérisée en ce que** les composants huileux englobent des huiles et/ou graisses végétales, animales, minérales ou synthétiques, des huiles de silicones volatiles et/ou non-volatiles et des cires.

12. Masse géliforme selon la revendication 11, **caractérisée en ce que** l'huile végétale, animale, minérale ou synthétique et/ou l'huile de silicone volatile ou non-volatile représentent de 1 à 70% en poids du poids total de la composition .

13. Masse géliforme selon la revendication 12, **caractérisée en ce que** l'huile de ricin, l'huile de tournesol, l'huile de sésame, l'huile de colza, l'huile de coco hydrogénée, l'huile de palme hydrogénée, l'huile de jojoba, l'huile minérale, la paraffine, la vaseline, la lanoline ou autres dérivés de la lanoline, l'isoparaffine volatile, le cyclométhicone, le diméthicone, le phényltriméthicone, ou un mélange des huiles précitées sont utilisables en tant qu'huile et/ou graisse.

14. Masse géliforme selon l'une quelconque des revendications précédentes, **caractérisée en ce que** la cire est présente à raison de 0,1 à 30% en poids par rapport au poids total de la composition.

15. Masse géliforme selon la revendication 14, **caractérisée en ce qu'**elle contient, comme cire, une cire naturelle, minérale ou synthétique.

16. Masse géliforme selon la revendication 15, **caractérisée en ce qu'**elle contient, comme cire, de la cire d'abeilles, de la cire d'abeilles modifiée (dite « Cera Bellina »), de la cire de Carnauba, de la cire de Candelilla, de la cire du Japon, de la cire d'Ouricouri, de la cire florale, de la cire de fleur d'oranger, de la cire de Montana, de la cire microcristalline, des alcools gras à longue chaîne, des esters d'alcools gras à longue chaîne avec des acides gras à longue chaîne ou des mélanges des cires précitées.

17. Masse géliforme selon l'une quelconque des revendications précédentes, **caractérisée en ce que** l'alcool gras à longue chaîne est l'alcool cétylique, l'alcool stéarylique, l'alcool béhènylique ou un de leurs mélanges.

18. Masse géliforme selon l'une quelconque des revendications précédentes, **caractérisée en ce que** l'ester d'alcool gras à longue chaîne et d'acide gras à longue chaîne est le palmitate de cétyle, le palmitate de cétéaryle, le stéarate de stéaryle, le stéarate de béhènyle, un stéarate d'alkyle en C 20-40 ou un de leurs mélanges.

19. Masse géliforme selon l'une quelconque des revendications précédentes, **caractérisée en ce qu'**elle contient des agents adjuvants et additionnels, à raison de 0,1 à 50% en poids du poids total de la composition.

20. Masse géliforme selon l'une quelconque des revendications précédentes, **caractérisée en ce qu'**elle contient des pigments à raison de 0,1 à 50% en poids, de préférence de 1 à 45% en poids et plus préférentiellement de 5 à 35% en poids du poids total de la composition.

21. Masse géliforme selon l'une quelconque des revendications précédentes, **caractérisée en ce qu'**elle contient, comme pigments, du dioxyde de titane, de l'oxyde de fer, du bleu outremer, du vert d'oxyde de chrome, du vert d'oxyde de chrome hydraté, du bleu de Berlin, du mica, des pigments brillants, de la poudre métallique en forme de plaquettes, des plaquettes de PET finement divisées, des colorants organiques pour laques, ou leurs mélanges.

22. Masse géliforme selon l'une quelconque des revendications précédentes, **caractérisée en ce qu'**elle se présente sous forme d'une mine coulée ou extrudée.

23. Crayon de couleur comportant une mine réalisée en une masse géliforme selon l'une quelconque des revendications 1 à 22, entourée d'une gaine en bois, en un matériau de remplacement du bois, ou en matière plastique.

24. Crayon de couleur comportant une mine réalisée en une masse géliforme selon l'une quelconque des revendications 1 à 22, insérée ou coulée dans le mécanisme de rotation d'un crayon rotatif.

25. Crayon de couleur selon l'une ou l'autre des revendications 23 ou 24, **caractérisé en ce qu'**il s'agit d'un crayon cosmétique.

26. Crayon de couleur selon l'une ou l'autre des revendications 23 ou 24, **caractérisé en ce qu'**il s'agit d'un bâton pour les lèvres, un crayon pour le contour des lèvres, un crayon pour les paupières, un eyeliner, un applicateur de khol, de kajal, d'ombre à paupières ou encore d'un crayon de protection ( dit « Concealer » ou anti-cernes).

27. Procédé pour la fabrication d'un crayon de couleur, **caractérisé en ce qu'**il consiste à fondre une masse géliforme selon l'une quelconque des revendications 1 à 22, puis à la couler dans une gaine pour crayon à mine, en bois, en succédané de bois, ou en matière plastique, et enfin à l'usiner en un crayon.

28. Procédé pour la fabrication d'un crayon de couleur, **caractérisé en ce qu'**il consiste à extruder une masse géliforme selon l'une quelconque des revendications 1 à 22, à l'allonger en portions de mines pour crayons, à la coller dans des planchettes mortaisées en bois, en succédané de bois ou en matière plastique, et enfin, à l'usiner en un crayon.

29. Procédé pour la fabrication d'un crayon de couleur, **caractérisé en ce qu'**il consiste, pour l'obtention d'une mécanique à rotation, à fondre une masse géliforme selon l'une quelconque des revendications 1 à 22, à la couler à travers un porte-mines d'une mécanique à rotation adaptée à un moule de coulée, et, après refroidissement et démoulage de la mine obtenue, à l'insérer par rotation dans la mécanique à rotation un crayon rotatif.

30. Procédé pour la fabrication d'un crayon de couleur, **caractérisé en ce qu'**il consiste, pour l'obtention d'une mécanique à rotation, à fondre une masse géliforme selon l'une quelconque des revendications 1 à 22, à la couler dans un moule de coulée, et, après refroidissement de la mine obtenue, à l'insérer dans le porte-mines d'un mécanisme à rotation d'un crayon rotatif.
